# EUROPEAN PATENT SPECIFICATION

(11) **EP 2 388 340 B1**
(45) Date of publication and mention of the grant of the patent: **03.04.2013**
(21) Application number: 11006740.2
(22) Date of filing: 26.10.2005
(51) Int. Cl.: C12Q 1/70, C12N 15/09, C12Q 1/68

(54) **Method of detecting H7 avian influenza virus**
Verfahren zum Nachweisen von H7-Geflügelpestvirus
Procédé de détection de virus de la grippe aviaire H7

(30) Priority: 01.11.2004 JP 2004318214; 20.05.2005 JP 2005148487
(43) Date of publication of application: 23.11.2011
(62) Divisional of application: 05805278.8
(73) Proprietor: EIKEN KAGAKU KABUSHIKI KAISHA, Tokyo 110-8408 (JP); JAPAN as represented by DIRECTOR GENERAL OF NATIONAL INSTITUTE OF INFECTIOUS DISEASES, Tokyo 162-8640 (JP)
(72) Inventor: Minekawa, Harumi c/o Nasu Kojo, Eiken Kagaru Kabushiki Kaisha, Otawara-shi, Tochigi 324-0036 (JP); Notomi, Tsugunori c/o Nasu Kojo, Eiken Kagaru Kabushiki Kaisha, Otawara-shi, Tochigi 324-0036 (JP); Yonekawa, Toshihiro c/o Nasu Kojo, Eiken Kagaru Kabushiki Kaisha, Otawara-shi, Tochigi 324-0036 (JP); Tomita, Norihiro c/o Nasu Kojo, Eiken Kagaru Kabushiki Kaisha, Otawara-shi, Tochigi 324-0036 (JP); Kuzuhara, Yoko c/o Nasu Kojo, Eiken Kagaru Kabushiki Kaisha, Otawara-shi, Tochigi 324-0036 (JP); Odagiri, Takato c/o Murayama Branch, Musashimurayama-shi, Tokyo 208-0011 (JP)
(74) Representative: Grünecker, Kinkeldey, Stockmair & Schwanhäusser

(56) References cited:
- EP-A- 1 020 534
- LAU L-T ET AL: "Nucleic acid sequence-based amplification methods to detect avian influenza virus", BIOCHEMICAL AND BIOPHYSICAL RESEARCH COMMUNICATIONS, ACADEMIC PRESS INC. ORLANDO, FL, US, vol. 313, no. 2, 9 January 2004 (2004-01-09), pages 336-342, XP004601915, ISSN: 0006-291X
- COLLINS RICHARD A ET AL: "Rapid and sensitive detection of avian influenza virus subtype H7 using NASBA.", BIOCHEMICAL AND BIOPHYSICAL RESEARCH COMMUNICATIONS, vol. 300, no. 2, 10 January 2003 (2003-01-10), pages 507-515, XP002534229, ISSN: 0006-291X
- THAI H T C ET AL: "Development and evaluation of a novel loop-mediated isothermal amplification methods for rapid detection of severe acute respiratory syndrome coronavirus", JOURNAL OF CLINICAL MICROBIOLOGY, WASHINGTON, DC, US, vol. 42, no. 5, 1 May 2004 (2004-05-01), pages 1956-1961, XP002981787, ISSN: 0095-1137
- YOSHIKAWA TETSUSHI ET AL: "Detection of human herpesvirus 7 DNA by loop-mediated isothermal amplification.", JOURNAL OF CLINICAL MICROBIOLOGY MAR 2004, vol. 42, no. 3, March 2004 (2004-03), pages 1348-1352, XP002534230, ISSN: 0095-1137
- PARIDA MANMOHAN ET AL: "Real-time reverse transcription loop-mediated isothermal amplification for rapid detection of West Nile virus.", JOURNAL OF CLINICAL MICROBIOLOGY JAN 2004, vol. 42, no. 1, January 2004 (2004-01), pages 257-263, XP002534231, ISSN: 0095-1137
- NOTOMI T ET AL: "Loop-mediated isothermal amplification of DNA", NUCLEIC ACIDS RESEARCH, OXFORD UNIVERSITY PRESS, SURREY, GB, vol. 28, no. 12, 1 December 2000 (2000-12-01), pages I-VII, XP007905272, ISSN: 0305-1048
- IMAI M ET AL: "Development of H5-RT-LAMP (loop-mediated isothermal amplification) system for rapid diagnosis of H5 avian influenza virus infection", VACCINE, BUTTERWORTH SCIENTIFIC. GUILDFORD, GB, vol. 24, no. 44-46, 10 November 2006 (2006-11-10), pages 6679-6682, XP025152002, ISSN: 0264-410X [retrieved on 2006-11-10]

### SEQUENCE LISTING

<110> Eiken Kagaku Kabushiki Kaisha Japan as represented by Director General of National Institute of Infectious Diseases
<120> METHOD OF DETECTING H5 OR H7 AVIAN INFLUENZA VIRUS
<130> EP51595IHVTRpau
<140> not yet assigned
   <141> herewith
<150> EP 05 805 278.8
   <151> 2005-10-26
<150> PCT/JP2005/019710
   <151> 2005-10-26
<150> JP2005-148487
   <151> 2005-05-20
<150> JP2004-318214
   <151> 2004-11-01
<160> 59
<170> PatentIn version 3.1
<210> 1
   <211> 1674
   <212> DNA
   <213> Avian influenza virus
<400> 1
   agtcttgtta aaagtgatca gatttgcatt ggttaccatg caaacaactc gacagagcag 60
   gttgacacaa taatggaaaa gaacgttact gttacacatg cccaagacat attggaaaag 120
   acacacaatg ggaagctctg cgatctagat ggagtgaagc ctctaatttt gagagattgt 180
   agtgtagctg gatggctcct cggaaaccca atgtgtgacg aattcatcaa tgtgccggaa 240
   tggtcttaca tagtggagaa ggccagtcca gccaatgacc tctgttaccc aggggatttc 300
   aacgactatg aagaactgaa acacctattg agcagaataa accattttga gaaaattcag 360
   atcatcccca aaagttcttg gtccaatcat gaagcctcat caggggtgag ctcagcatgt 420
   ccatatcttg ggaagtcctc ctttttcaga aatgtggtat ggcttatcaa aaagaacagt 480
   acatacccaa caataaagag gagctataat aataccaacc aagaagatct tttggtactg 540
   tgggggattc accatcctaa tgatgcggca gagcagacaa agctctatca aaacccaacc 600
   acctatattt ccgttggaac atcaacacta aaccagagat tggtaccaaa aatagctact 660
   agatccaaag taaacgggca aagtggaaga atggagttct tctggacaat tttaaagccg 720
   aatgatgcta tcaatttcga gagtaatgga aatttcattg ctccagaata tgcatacaaa 780
   attgtcaaga aaggggactc agcaattatg aaaagtgaat tggaatatgg taactgcaac 840
   accaagtgtc aaactccaat gggggcgata aactctagta tgccattcca caacatacac 900
   cctctcacca tcggggaatg ccccaaatat gtgaaatcaa acagattagt ccttgcgact 960
   ggactcagaa atacccctca aagagagaga agaagaaaaa agagaggact atttggagct 1020
   atagcaggtt ttatagaggg aggatggcag ggaatggtag atggttggta tgggtaccac 1080
   catagcaatg agcaggggag tggatacgct gcagacaaag aatccactca aaaggcaata 1140
   gatggagtta ccaataaggt caactcgatc attgacaaaa tgaacactca gtttgaggcc 1200
   gttggaaggg aatttaataa cttagaaagg agaatagaaa atttaaacaa gaagatggaa 1260
   gacggattcc tagatgtctg gacttataat gctgaacttc tggttctcat ggaaaatgag 1320
   agaactctag actttcacga ctcaaatgtc aagaaccttt acgacaaggt ccgactacag 1380
   cttagggata atgcaaagga gctgggtaac ggctgtttcg agttctatca caaatgtgat 1440
   aatgaatgta tggaaagtgt aaaaaacgga acgtatgact acccgcagta ttcagaagaa 1500
   gcaagactaa acagagagga aataagtgga gtaaaattgg aatcaatggg aacttaccaa 1560
   atactgtcaa tttattcaac agtggcgagt tccctagcac tggcaatcat ggtagctggt 1620
   ctatctttat ggatgtgctc caatggatcg ttacaatgca gaatttgcat ttaa 1674
<210> 2
   <211> 28
   <212> DNA
   <213> Artificial
<220>
   <223> primer
<400> 2
   aattatgaaa agtgagttgg aatatggt 28
<210> 3
   <211> 19
   <212> DNA
   <213> Artificial
<220>
   <223> primer
<400> 3
   tcattgctcc agaatatgc 19
<210> 4
   <211> 17
   <212> DNA
   <213> Artificial
<220>
   <223> primer
<400> 4
   ggagttcttc tggacaa 17
<210> 5
   <211> 17
   <212> DNA
   <213> Artificial
<220>
   <223> primer
<400> 5 17
   caaactccaa tgggggc 17
<210> 6
   <211> 17
   <212> DNA
   <213> Artificial
<220>
   <223> primer
<400> 6
   atacaccctc tcaccat 17
<210> 7
   <211> 17
   <212> DNA
   <213> Artificial
<220>
   <223> primer
<400> 7
   agattagtcc ttgcgac 17
<210> 8
   <211> 47
   <212> DNA
   <213> Artificial
<220>
   <223> primer
<400> 8
   accatattcc aactcacttt tcataatttc attgctccag aatatgc 47
<210> 9
   <211> 34
   <212> DNA
   <213> Artificial
<220>
   <223> primer
<400> 9
   caaactccaa tgggggcatg gtgagagggt gtat 34
<210> 10
   <211> 17
   <212> DNA
   <213> Artificial
<220>
   <223> primer
<400> 10
   gtcgcaagga ctaatct 17
<210> 11
   <211> 20
   <212> DNA
   <213> Artificial
<220>
   <223> primer
<400> 11
   gagtcccctt tcttgacaat 20
<210> 12
   <211> 19
   <212> DNA
   <213> Artificial
<220>
   <223> primer
<400> 12
   gataaactct agtatgcca 19
<210> 13
   <211> 22
   <212> DNA
   <213> Artificial
<220>
   <223> primer
<400> 13
   aacgttactg ttacacatgc cc 22
<210> 14
   <211> 18
   <212> DNA
   <213> Artificial
<220>
   <223> primer
<400> 14
   aaacaactcg acagagca 18
<210> 15
   <211> 20
   <212> DNA
   <213> Artificial
<220>
   <223> primer
<400> 15
   cagatttgca ttggttacca 20
<210> 16
   <211> 22
   <212> DNA
   <213> Artificial
<220>
   <223> primer
<400> 16
   tggaaaagac acacaatggg aa 22
<210> 17
   <211> 20
   <212> DNA
   <213> Artificial
<220>
   <223> primer
<400> 17
   gattgtagtg tagctggatg 20
<210> 18
   <211> 18
   <212> DNA
   <213> Artificial
<220>
   <223> primer
<400> 18
   gaaacccaat gtgtgacg 18
<210> 19
   <211> 40
   <212> DNA
   <213> Artificial
<220>
   <223> primer
<400> 19
   gggcatgtgt aacagtaacg ttaaacaact cgacagagca 40
<210> 20
   <211> 42
   <212> DNA
   <213> Artificial
<220>
   <223> primer
<400> 20
   tggaaaagac acacaatggg aacatccagc tacactacaa tc 42
<210> 21
   <211> 18
   <212> DNA
   <213> Artificial
<220>
   <223> primer
<400> 21
   cgtcacacat tgggtttc 18
<210> 22
   <211> 19
   <212> DNA
   <213> Artificial
<220>
   <223> primer
<400> 22
   ttccattatt gtgtcaacc 19
<210> 23
   <211> 20
   <212> DNA
   <213> Artificial
<220>
   <223> primer
<400> 23
   cgatctagat ggagtgaagc 20
<210> 24
   <211> 20
   <212> DNA
   <213> Artificial
<220>
   <223> primer
<400> 24
   ctcctcggaa acccaatgtg 20
<210> 25
   <211> 19
   <212> DNA
   <213> Artificial
<220>
   <223> primer
<400> 25
   atctagatgg agtgaagcc 19
<210> 26
   <211> 19
   <212> DNA
   <213> Artificial
<220>
   <223> primer
<400> 26 19
   ggaaaagaca cacaatggg 19
<210> 27
   <211> 21
   <212> DNA
   <213> Artificial
<220>
   <223> primer
<400> 27
   ttcatcaatg tgccggaatg g 21
<210> 28
   <211> 18
   <212> DNA
   <213> Artificial
<220>
   <223> primer
<400> 28
   tacccagggg atttcaac 18
<210> 29
   <211> 20
   <212> DNA
   <213> Artificial
<220>
   <223> primer
<400> 29
   aactgaaaca cctattgagc 20
<210> 30
   <211> 39
   <212> DNA
   <213> Artificial
<220>
   <223> primer
<400> 30
   cacattgggt ttccgaggag atctagatgg agtgaagcc 39
<210> 31
   <211> 39
   <212> DNA
   <213> Artificial
<220>
   <223> primer
<400> 31
   ttcatcaatg tgccggaatg ggttgaaatc ccctgggta 39
<210> 32
   <211> 20
   <212> DNA
   <213> Artificial
<220>
   <223> primer
<400> 32
   gctcaatagg tgtttcagtt 20
<210> 33
   <211> 20
   <212> DNA
   <213> Artificial
<220>
   <223> primer
<400> 33
   ccagctacac tacaatctct 20
<210> 34
   <211> 18
   <212> DNA
   <213> Artificial
<220>
   <223> primer
<400> 34
   tccagccaat gacctctg 18
<210> 35
   <211> 22
   <212> DNA
   <213> Artificial
<220>
   <223> primer
<400> 35 22
   tcaaacagat tagtccttgc ga 22
<210> 36
   <211> 18
   <212> DNA
   <213> Artificial
<220>
   <223> primer
<400> 36
   catacaccct ctcaccat 18
<210> 37
   <211> 20
   <212> DNA
   <213> Artificial
<220>
   <223> primer
<400> 37 20
   tctagtatgc cattccacaa 20
<210> 38
   <211> 22
   <212> DNA
   <213> Artificial
<220>
   <223> primer
<400> 38 22
   tacccctcaa agagagagaa ga 22
<210> 39
   <211> 20
   <212> DNA
   <213> Artificial
<220>
   <223> primer
<400> 39
   caggttttat agagggagga 20
<210> 40
   <211> 18
   <212> DNA
   <213> Artificial
<220>
   <223> primer
<400> 40
   cagggaatgg tagatggt 18
<210> 41
   <211> 40
   <212> DNA
   <213> Artificial
<220>
   <223> primer
<400> 41
   tcgcaaggac taatctgttt gacatacacc ctctcaccat 40
<210> 42
   <211> 42
   <212> DNA
   <213> Artificial
<220>
   <223> primer
<400> 42
   tacccctcaa agagagagaa gatcctccct ctataaaacc tg 42
<210> 43
   <211> 18
   <212> DNA
   <213> Artificial
<220>
   <223> primer
<400> 43
   accatctacc attccctg 18
<210> 44
   <211> 20
   <212> DNA
   <213> Artificial
<220>
   <223> primer
<400> 44
   tcacatattt ggggcattcc 20
<210> 45
   <211> 20
   <212> DNA
   <213> Artificial
<220>
   <223> primer
<400> 45
   agagaggact atttggagct 20
<210> 46
   <211> 20
   <212> DNA
   <213> Artificial
<220>
   <223> RT-PCR primer (H5 515f)
<400> 46
   catacccaac aataaagagg 20
<210> 47
   <211> 20
   <212> DNA
   <213> Artificial
<220>
   <223> RT-PCR primer (H5 1220r)
<400> 47
   gtgttcattt tgttaatgat 20
<210> 48
   <211> 1737
   <212> DNA
   <213> Avian influenza virus
<400> 48
   agcaaaagca ggggatacaa aatgaacact caaatcctgg tattcgctct ggtggcgagc 60
   attccgacaa atgcagacaa gatctgcctt gggcatcatg ccgtgtcaaa cgggactaaa 120
   gtaaacacat taactgagag aggagtggaa gtcgttaatg caactgaaac ggtggaacga 180
   acaaacgttc ccaggatctg ctcaaaaggg aaaaggacag ttgacctcgg tcaatgtgga 240
   cttctgggaa caatcactgg gccaccccaa tgtgaccaat tcctagaatt ttcggccgac 300
   ttaattattg agaggcgaga aggaagtgat gtctgttatc ctgggaaatt cgtgaatgaa 360
   gaagctctga ggcaaattct cagagagtca ggcggaattg acaaggagac aatgggattc 420
   acctacagcg gaataagaac taatggaaca accagtgcat gtaggagatc aggatcttca 480
   ttctatgcag agatgaaatg gctcctgtca aacacagaca atgctgcttt cccgcaaatg 540
   actaagtcat acaagaacac aaggaaagac ccagctctga taatatgggg gatccaccat 600
   tccggatcaa ctacagaaca gaccaagcta tatgggagtg gaaacaaact gataacagtt 660
   gggagttcta attaccaaca gtcctttgta ccgagtccag gagcgagacc acaagtgaat 720
   ggccaatctg gaagaattga ctttcattgg ctgatactaa accctaatga cacggtcact 780
   ttcagtttca atggggcctt catagctcca gaccgtgcaa gctttctgag agggaagtcc 840
   atgggaattc agagtgaagt acaggttgat gccaattgtg aaggagattg ctatcatagt 900
   ggagggacaa taataagtaa tttgcccttt cagaacataa atagcagggc agtaggaaaa 960
   tgtccgagat atgttaagca agagagtctg ctgttggcaa caggaatgaa gaatgttccc 1020
   gaaatcccaa agaggaggag gagaggccta tttggtgcta tagcgggttt cattgaaaat 1080
   ggatgggaag gtttgattga tgggtggtat ggcttcaggc atcaaaatgc acaaggggag 1140
   ggaactgctg cagattacaa aagcacccaa tcagcaattg atcaaataac agggaaatta 1200
   aatcggctta tagaaaaaac taaccaacag tttgagttaa tagacaacga attcactgag 1260
   gttgaaaggc aaattggcaa tgtgataaac tggaccagag attccatgac agaagtgtgg 1320
   tcctataacg ctgaactctt agtagcaatg gagaatcagc acacaattga tctggccgac 1380
   tcagaaatga acaaactgta cgaacgagtg aagagacaac tgagagagaa tgccgaagaa 1440
   gatggcactg gttgcttcga aatatttcac aagtgtgatg acgactgcat ggccagtatt 1500
   agaaacaaca cctatgatca cagcaagtac agggaagaag caatacaaaa tagaatacag 1560
   attgacccag tcaaactaag cagcggctac aaagatgtga tactttggtt tagcttcggg 1620
   gcatcatgtt tcatacttct ggccattgca atgggccttg tcttcatatg tgtgaagaat 1680
   ggaaacatgc ggtgcactat ttgtatataa gtttggaaaa acacccttgt ttctact 1737
<210> 49
   <211> 21
   <212> DNA
   <213> Artificial
<220>
   <223> primer
<400> 49
   aaggtttgat tgatgggtgg t 21
<210> 50
   <211> 19
   <212> DNA
   <213> Artificial
<220>
   <223> primer
<400> 50
   ctatttggtg ctatagcgg 19
<210> 51
   <211> 16
   <212> DNA
   <213> Artificial
<220>
   <223> primer
<400> 51
   ttcccgaaat cccaaa 16
<210> 52
   <211> 22
   <212> DNA
   <213> Artificial
<220>
   <223> primer
<400> 52
   ttcaggcatc aaaatgcaca ag 22
<210> 53
   <211> 22
   <212> DNA
   <213> Artificial
<220>
   <223> primer
<400> 53 22
   cagcaattga tcaaataaca gg 22
<210> 54
   <211> 22
   <212> DNA
   <213> Artificial
<220>
   <223> primer
<400> 54 22
   cggcttatag aaaaaactaa cc 22
<210> 55
   <211> 40
   <212> DNA
   <213> Artificial
<220>
   <223> primer
<400> 55
   accacccatc aatcaaacct tctatttggt gctatagcgg 40
<210> 56
   <211> 44
   <212> DNA
   <213> Artificial
<220>
   <223> primer[
<400> 56
   ttcaggcatc aaaatgcaca agcctgttat ttgatcaatt gctg 44
<210> 57
   <211> 22
   <212> DNA
   <213> Artificial
<220>
   <223> primer
<400> 57 22
   ggttagtttt ttctataagc cg 22
<210> 58
   <211> 21
   <212> DNA
   <213> Artificial
<220>
   <223> primer
<400> 58
   cccatccatt ttcaatgaaa c 21
<210> 59
   <211> 20
   <212> DNA
   <213> Artificial
<220>
   <223> primer
<400> 59
   actgctgcag attacaaaag 20

## Description

### Technical Field

The present invention relates to an oligonucleotide primer set for detection of an H7 avian influenza virus, a method for detection of an H7 avian influenza virus using the primer set, a method for influenza diagnosis, and a kit for influenza diagnosis.

### Background Art

Influenza, which is an epidemic viral respiratory infection, affects people of the wide age bracket from infants to the old aged and is often fatal. Currently controversial H5 avian influenza viruses that infect birds do not originally infect humans. However, human infection with the viruses was confirmed in Hong Kong in 1997 and was prevalent with the result that 6 of 18 patients died. Fortunately, no human infection had been confirmed thereafter. However, human infection was confirmed in Thailand and Vietnam in 2004 with the result that 8 and 16 persons died in Thailand and Vietnam, respectively.

Highly pathogenic avian influenza viruses include an H7 subtype, in addition to the H5 subtype. The H7 subtypes are broadly divided into Eurasian and American subtypes according to the sequences thereof. According to reports, the H7 avian influenza viruses killed 1 person when prevalent in Netherlands in 2003, and were also prevalent in USA from 2003 through 2004.

A kit for quick diagnosis of a human influenza virus A has been used currently in the detection of avian influenza viruses. However, the identification of a virus subtype that causes infection has required further detailed analysis such as the antigenic analysis or genetic test of separated viruses.

Diagnosis using virus separation and culture that produces accurate results requires several days and therefore, cannot be conducted quickly. There are several methods capable of quick diagnosis as compared with the virus separation. Among them, an RT-PCR method has been said to have high detection sensitivity as compared with other methods. However, according to some reports, currently disclosed RT-PCR methods cannot detect the viruses with high sensitivity as compared with viral infectivity. Thus, infection with an avian influenza virus cannot be denied even if a result is negative in a test using the RT-PCR method.

Thus, a test method capable of quickly detecting an H5 or H7 avian influenza virus with high sensitivity has been demanded.

Patent Document 1: European Patent Publication No. 1310565
Patent Document 2: Japanese Published PCT Translation No. 2004-509648
Patent Document 3: EP-A-1 020 534
Non-Patent Document 1: Lau LT., et al., Biochem. Biophys. Res. Commun., vol. 313, p. 336-342 (2004)
Non-Patent Document 2: Shan S., et al., Biochem. Biophys. Res. Commun., vol. 302, p. 377-383 (2003)
Non-Patent Document 3: Collins RA., et al., Biochem. Biophys. Res. Commun., vol. 300, p. 507-515 (2003)
Non-Patent Document 4: Lee MS., et al., J. Virol. Methods, vol. 97, p. 13-22 (2001)
Non-Patent Document 5: Munch M., et al., Arch. Virol., vol. 146, p. 87-97(2001)
Non-Patent Document 6: THAI H T C ET AL. "Development and evaluation of a novel loop-mediated isothermal amplification methods for rapid detection of severe acute respiratory syndrome coronavirus" JOURNAL OF CLINICAL MICROBIOLOGY, vol. 42, no. 5, 1 May 2004 (2004-05-01), pages 1956-1961.
Non-Patent Document 7: YOSHIKAWA TETSUSHI ET AL. "Detection of human herpesvirus 7 DNA by loop-mediated isothermal amplification." JOURNAL OF CLINICAL MICROBIOLOGY; vol. 42, no. 3, March 2004 (2004-03), pages 1348-1352.
Non-Patent Document 8: PARIDA MANMOHAN ET AL: "Real-time reverse transcription loop-mediated isothermal amplification for rapid detection of West Nile virus." JOURNAL OF CLINICAL MICROBIOLOGY, vol. 42, no. 1, January 2004 (2004-01), pages 257-263.
Non-Patent Document 9: NOTOMI T ET AL. "Loop-mediated isothermal amplification of DNA" NUCLEIC ACIDS RESEARCH, vol. 28, no. 12, 1 December 2000 (2000-12-01), pages i-vii.
Non-Patent Documents 1 and 3 refer to the detection of avian influenza H5 or H7 viruses based on the amplification of a part of the haemagglutinin gene as the target gene.

### Disclosure of the Invention

### Problems to be Solved by the Invention

The present inventors have conducted diligent studies for solving the problems and have consequently completed the present invention by finding out that an H7 avian influenza virus can be detected with high sensitivity by preparing oligonucleotide primers hybridizing to a nucleotide sequence specific to the H7 avian influenza virus and amplifying the nucleotide sequence specific to the H7 avian influenza virus by a LAMP (loop-mediated isothermal amplification) method.

### Means for Solving the Problems

Specifically, the present invention provides the following (1) to (6):
(1) An oligonucleotide primer set, characterized by being capable of amplifying a nucleotide sequence specific for the H7 avian influenza virus and consisting of an inner primer set comprising oligonucleotide primers comprising the following a) and b), and, an outer primer set comprising oligonucleotide primers comprising the following c) and d):
   a) 5'-(a nucleotide sequence complementary to the nucleotide sequence of SEQ ID NO: 49)-(an arbitrary nucleotide sequence having 0 to 50 bases)-(the nucleotide sequence of SEQ ID NO: 50)-3';
   b) 5'-(the nucleotide sequence of SEQ ID NO: 52)-(an arbitrary nucleotide sequence having 0 to 50 bases)-(a nucleotide sequence complementary to the nucleotide sequence of SEQ ID NO: 53)-3';
   c) a nucleotide sequence of SEQ ID NO: 51; and
   d) a nucleotide sequence of SEQ ID NO: 57.
(2) The oligonucleotide primer set according to (1), wherein the inner primer set comprises oligonucleotide primers comprising the nucleotide sequence of SEQ ID NOs: 55 and 56.
(3) The oligonucleotide primer set according to (2) further comprising a loop primer set comprising oligonucleotide primers comprising the nucleotide sequence of SEQ ID NOs: 58 and 59.
(4) A method for detection of an H7 avian influenza virus, characterized by comprising performing an amplification reaction of a target nucleic acid region of an H7 avian influenza virus by use of an oligonucleotide primer set according to any one of (1) to (3), wherein the amplification reaction is a LAMP method.
(5) A method for influenza diagnosis, characterized by comprising detecting amplification of a target nucleic acid region of an H7 avian influenza virus by use of an oligonucleotide primer set according to any one of (1) to (3) and thereby diagnosing the presence or absence of infection with the H7 avian influenza virus.
(6) A kit for influenza diagnosis, characterized by comprising an oligonucleotide primer set according to any one of (1) to (3).

### Effect of the Invention

According to the present invention, an H7 avian influenza virus can be detected quickly with high sensitivity by preparing oligonucleotide primers selectively hybridizing to a nucleotide sequence specific to the H7 avian influenza virus and amplifying the nucleotide sequence specific to the H7 avian influenza virus by a LAMP method.

### Brief Description of the Drawings

Figure 1 is a graph showing a result of a reactivity confirmation test of a primer set for an H7 avian influenza virus. 250, 100, and 50 represent the amounts of RNA added (copy/assay), and NC represents a negative control;
Figure 2 is a diagram showing a result of electrophoresis of products amplified with the primer set for an H7 avian influenza virus. Lane M represents a 100 bp ladder marker, Lane 1 represents a LAMP product sample, and Lane 2 represents a LAMP product sample treated with PstI;

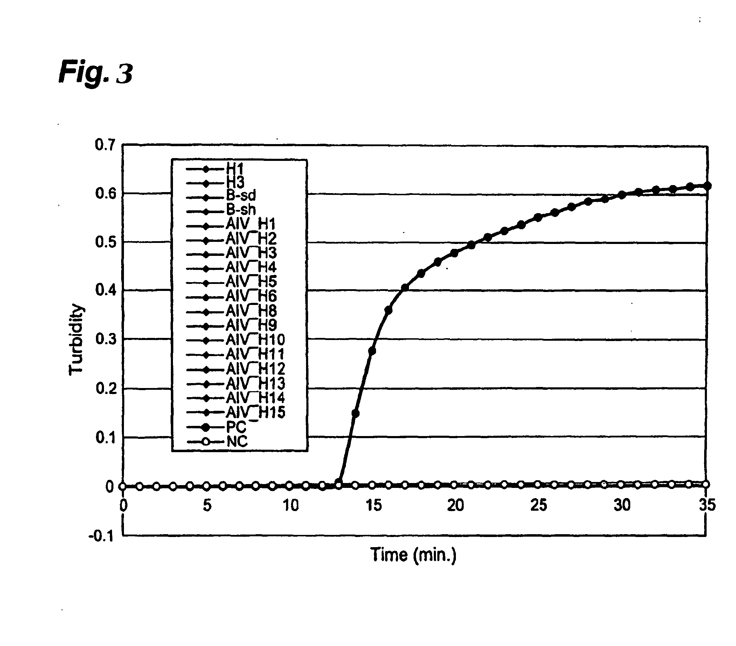

Figure 3 is a graph showing a result of a cross matching test of the primer set for an H7 avian influenza virus. H1 and H3 represent human influenza viruses H1 and H3, respectively, and B-sd represents B/Shandong/07/97, B-sh represents B/Shanghai/361/2002, AIV-H1 and so on represents avian influenza virus H1 and so on, PC represents a positive control, and NC represents a negative control;
Figure 4 is a graph showing a result of the reactivity of the primer set for an H7 avian influenza virus to various strains. Figure 4 (a) represents A/Netherlands/219/2003, and Figure 4 (b) represents A/Netherlands/33/2003. 10⁵ to 10⁸ represent dilution rates of RNA extracts; and
Figure 5 is a graph showing a result of the reactivity of the primer set for an H7 avian influenza virus to various strains. Figure 5 (a) represents A/mallard/Netherlands/12/00, and Figure 5 (b) represents A/wigeon/Osaka/1/2001. 10⁵ to 10⁸ represent dilution rates of RNA extracts.

### Best Mode for Carrying Out the Invention

A sample used in the present invention includes samples derived from human or other animal living bodies suspected of being infected with an influenza virus, for example, sputum, bronchoalveolar lavage fluids, nasal secretions, nasal aspirates, nasal lavage fluids, nasal swabs, pharyngeal swabs, throat washings, saliva, blood, serums, plasmas, spinal fluids, urine, feces, and tissues. Alternatively, cells or culture solutions thereof used in infection experiments or the like, or virus-containing samples separated from living body-derived samples or cultured cells may also be used as the sample. These samples may be subjected to pretreatment such as separation, extraction, condensation, and purification.

Such nucleic acid amplification is achieved by a loop-mediated isothermal amplification method called a LAMP method developed by Notomi et al., which is a novel nucleic acid amplification method that does not require temperature control allegedly indispensable for PCR methods (Pamphlet of International Publication No. WO 00/28082). This method is a nucleic acid amplification method that allows for a complementary strand synthesis reaction under isothermal conditions by allowing a nucleotide serving as a template to anneal with its own 3' end and initiating complementary strand synthesis from this origin while combining primers annealing to this formed loop. Moreover, the LAMP method is a highly specific nucleic acid amplification method using 4 primers that recognize at least 6 regions.

Oligonucleotide primers used in the LAMP method are at least 4 primers that recognize the nucleotide sequences of 6 regions in total, that is, F3c, F2c, and F1c regions from the 3' end side and B3, B2, and B1 regions from the 5' end side, of the nucleotide sequence of a template nucleic acid, and are respectively called inner primers F and B and outer primers F and B. Complementary sequences of F1c, F2c, and F3c are called F1, F2, and F3, respectively. Complementary sequences of B1, B2, and B3 are called B1c, B2c, and B3c, respectively. The inner primer is an oligonucleotide having, at the 3' end, a nucleotide sequence that recognizes a "certain nucleotide sequence region" in a target nucleotide sequence and provides a synthesis origin and having, at the 5' end, a nucleotide sequence complementary to an arbitrary region of a nucleic acid synthesis reaction product obtained with this primer at the origin. In this context, a primer comprising a "nucleotide sequence selected from F2" and a "nucleotide sequence selected from F1c" is called an inner primer F (hereinafter, abbreviated to FIP), and a primer comprising a "nucleotide sequence selected from B2" and a "nucleotide sequence selected from B1c" is called an inner primer B (hereinafter, abbreviated to BIP). On the other hand, the outer primer is an oligonucleotide having a nucleotide sequence that recognizes a "certain nucleotide sequence region present nearer to the 3' end side than the regions recognized by the inner primers" in the target nucleotide sequence and provides a synthesis origin. In this context, a primer comprising a "nucleotide sequence selected from F3" is called an outer primer F (hereinafter, abbreviated to F3), and a primer comprising a "nucleotide sequence selected from B3" is called an outer primer B (hereinafter, abbreviated to B3). In this context, F in each primer indicates that the primer complementarily binds to the sense strand of the target nucleotide sequence and provides a synthesis origin. On the other hand, B in each primer indicates that the primer complementarily binds to the antisense strand of the target nucleotide sequence and provides a synthesis origin. In this context, the oligonucleotide used as the primer is 10 bases or more, preferably 15 bases or more, in length, and may be either synthesized chemically or natural. Each primer may be a single oligonucleotide or a mixture of several oligonucleotides.

In the LAMP method, additional primers, that is, loop primers, can further be used in addition to the inner and outer primers. The loop primers refer to 2 primers (one for each of strands composing a double-strand) comprising, at the 3' end, a nucleotide sequence complementary to a sequence in a loop formed by the annealing of complementary sequences present at the same strand of an amplification product obtained by the LAMP method. The use of the loop primers increases nucleic acid synthesis origins in number and achieves reduction in reaction time and enhancement in detection sensitivity (Pamphlet of International Publication No. WO 02/24902).

The oligonucleotide can be produced by a method known in the art and, for example, can be synthesized chemically. Alternatively, a natural nucleic acid is cleaved with a restriction enzyme or the like, and the resulting fragments may be modified or linked to compose a desired nucleotide sequence. Specifically, the oligonucleotide can be synthesized by use of an oligonucleotide synthesizer or the like. Alternatively, a production method known per se in the art can be used as a method for synthesis of an oligonucleotide comprising a nucleotide sequence with the substitution, deletion, insertion, or addition of one or several bases. For example, such an oligonucleotide may be synthesized by using site-specific mutagenesis, gene homologous recombination, primer extension, and PCR methods alone or in appropriate combination.

"Stringent hybridization conditions" used herein can be selected from those known generally. Examples of the stringent conditions include conditions involving overnight hybridization at 42°C in a solution containing 50% formamide, 5×SSC (150 mM NaCl, 15 mM trisodium citrate), 50 mM sodium phosphate (pH 7.6), 5×Denhardt's solution, 10% dextran sulfate, and 20 µg/ml DNA, followed by primary washing at room temperature in 2×SSC/0.1% SDS and subsequent secondary washing at approximately 65°C in 0.1×SSC/0.1% SDS.

Influenza viruses are RNA viruses. In the LAMP method using RNA as a template, a nucleic acid amplification reaction can be allowed to proceed in the same way as with template DNA by adding reverse transcriptase to a reaction solution for template DNA (RT-LAMP method).

The present inventors have conducted diligent studies on nucleotide sequences of primers of the LAMP method capable of quickly amplifying a nucleotide sequence specific to an H7 avian influenza virus and on combinations thereof and have consequently selected a primer set E described below from the nucleotide sequence of hemagglutinin of the H7 avian influenza virus (the nucleotide sequence represented by SEQ ID NO: 48).
(Primer set E)
22FIP: 5'-ACCACCCATCAATCAAACCTTCTATTTGGTGCTATAGCGG-3'
(SEQ ID NO: 55)
22BIP: 5'-TTCAGGCATCAAAATGCACAAGCCTGTTATTTGATCAATTGC TG-3' (SEQ ID NO: 56)
22F3m: 5'-TTCCCGAAATCCCAAA-3' (SEQ ID NO: 51)
22B3: 5'-GGTTAGTTTTTTCTATAAGCCG-3' (SEQ ID NO: 57)
22-9LF: 5'-CCCATCCATTTTCAATGAAAC-3' (SEQ ID NO: 58)
22-9LB: 5'-ACTGCTGCAGATTACAAAAG-3' (SEQ ID NO: 59)

An enzyme used in nucleic acid synthesis is not particularly limited as long as it is a template-dependent nucleic acid synthesis enzyme having strand displacement activities. Such an enzyme includes Bst DNA polymerase (large fragment), Bca (exo-) DNA polymerase, and the Klenow fragment of *E. coli* DNA polymerase I and preferably includes Bst DNA polymerase (large fragment).

Reverse transcriptase used in the RT-LAMP method is not particularly limited as long as it is an enzyme having activities of synthesizing cDNA with RNA as a template. Such an enzyme includes AMV, Cloned AMV, MMLV reverse transcriptases, Superscript II, ReverTra Ace, and Thermoscript and preferably includes AMV or Cloned AMV reverse transcriptase. Alternatively, the use of an enzyme having both reverse transcriptase and DNA polymerase activities, such as Bca DNA polymerase, can achieve an RT-LAMP reaction using a single enzyme.

The enzyme or reverse transcriptase used in nucleic acid synthesis may be purified from viruses, bacteria, or the like or may be prepared by a gene recombination technique. These enzymes may be modified by fragmentation, amino acid substitution, and so on.

A technique known in the art can be applied to the detection of nucleic acid amplification products after the LAMP reaction. For example, the nucleic acid amplification products can be detected by use of a labeled oligonucleotide specifically recognizing amplified nucleotide sequences or a fluorescent intercalator method (Japanese Patent Laid-Open No. 2001-242169), or can be detected easily by directly applying the reaction solution after the completion of reaction to agarose gel electrophoresis. In the agarose gel electrophoresis, the LAMP amplification products are detected in the form of a ladder of many bands differing in base length. Moreover, in the LAMP method, substrates are consumed in large amounts by nucleic acid synthesis, and pyrophosphoric acid ions as by-products are converted into magnesium pyrophosphate through its reaction with coexisting magnesium ions and makes the reaction solution cloudy to the extent that can be observed visually. Thus, the nucleic acid amplification reaction may be detected by confirming this cloudiness by use of a measurement apparatus that can optically observe time-dependent rises in turbidity after the completion of reaction or during reaction, for example, by confirming changes in absorbance at 400 nm by use of a usual spectrophotometer (Pamphlet of International Publication No. WO 01/83817).

A variety of reagents necessary for the detection of nucleic acid amplification by use of the primers of the present invention can be packaged in advance into a kit. Specifically, a kit provided comprises a variety of oligonucleotides necessary as the primers of the present invention or the loop primers, 4 dNTPs serving as substrates for nucleic acid synthesis, DNA polymerase for performing nucleic acid synthesis, an enzyme having reverse transcription activities, buffer solutions or salts that provide conditions suitable to an enzyme reaction, protective agents for stabilizing the enzymes or the templates and optionally comprises reagents necessary for the detection of reaction products.

### Examples

Hereinafter, the present invention will be described specifically with reference to Examples. However, the present invention is not limited to them by any means.

### Example 1: Confirmation of reactivity of primer set for H7 avian influenza virus

The reactivity of a primer set E was confirmed by a method described below. The composition of a reaction solution for nucleic acid amplification by a LAMP method is as described below. Primer synthesis was requested to QIAGEN, and the primers were used after OPC (reverse phase column cartridge) purification.
20 mM Tris-HCl pH 8.8
10 mM KCl
8 mM MgSO₄
1.4 mM dNTPs
10 mM (NH₄)₂SO₄
0.8 M Betaine (SIGMA)
0.1% Tween 20
1.6 µM FIP
1.6 µM BIP
0.2 µM F3
0.2 µM B3
0.8 µM LF
0.8 µM LB
AMV Reverse Transcriptase 2 U (Finnzyme)
Bst DNA polymerase 16 U (NEB)

The nucleotide sequence of A/Netherlands/219/2003 (H7N7) (strain separated from a death case in Netherlands) (a fragment comprising the nucleotide sequence represented by SEQ ID NO: 48) was incorporated into a cloning vector to prepare RNA through a transcription reaction. The prepared RNA was added at 250, 100, or 50 copies/assay to the reaction solution, and an amplification reaction and detection at 62.5°C for 35 minutes were performed by use of a real-time turbidity measurement apparatus LA-200 (Teramecs Co., Ltd.).

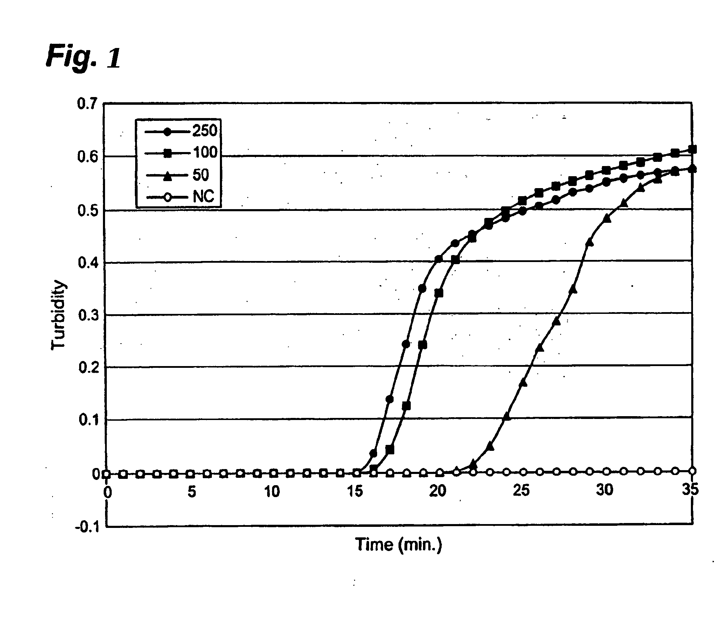

Figure 1 shows a result of real-time detection of 250, 100, and 50 copies/assay of RNA added and a negative control. This result demonstrated that up to 50 copies/assay are detected.

### Example 2: Confirmation of products amplified with primer set for H7 avian influenza virus

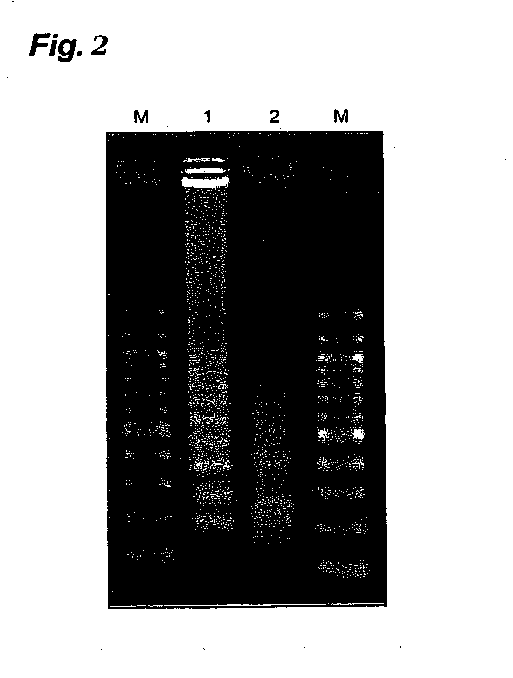

LAMP products amplified with the primer set E were confirmed by use of electrophoresis and a restriction enzyme PstI. Figure 2 is a graph showing a result of the electrophoresis. M represents a 100 bp ladder marker, Lane 1 represents an untreated LAMP product sample, and Lane 2 represents a LAMP product sample treated with PstI. As seen from the Lane 1 of Figure 2, a ladder pattern specific to the LAMP product was confirmed. Moreover, digestion was confirmed in the sample treated with PstI (Lane 2). These results revealed that the target sequence is specifically amplified.

### Example 3: Evaluation of primer set for H7 avian influenza virus (cross matching test)

To examine primer specificity, eighteen samples in total were used which included human influenza viruses A/New Caledonia/20/99 (H1N1), A/Panama/2007/99 (H3N2), B/Shangdong/07/97, and B/Shanghai/361/2002, and avian influenza viruses H1 to H15 (except for H7). RNA was extracted from each of the cultured viruses by use of QIAamp Viral RNA Kit (QIAGEN), and the extracted RNA was diluted 100 folds and used in an RT-LAMP reaction. The RNA prepared in Example 1 was used as a positive control (PC), and distilled water was used as a negative control.

As seen from Figu 3, amplification was not observed in any of the samples. These results revealed that the primer set of the present invention is highly specific.

### Example 4: Evaluation of primer set for H7 avian influenza virus (reactivity to various strains)

Four H7 avian influenza virus genomes were used as template samples to examine sensitivity to each virus. RNA extracted from each of the cultured viruses was serially diluted (10⁵ to 10⁸) with RNase-free sterilized water, and the 5 µL diluents were used.

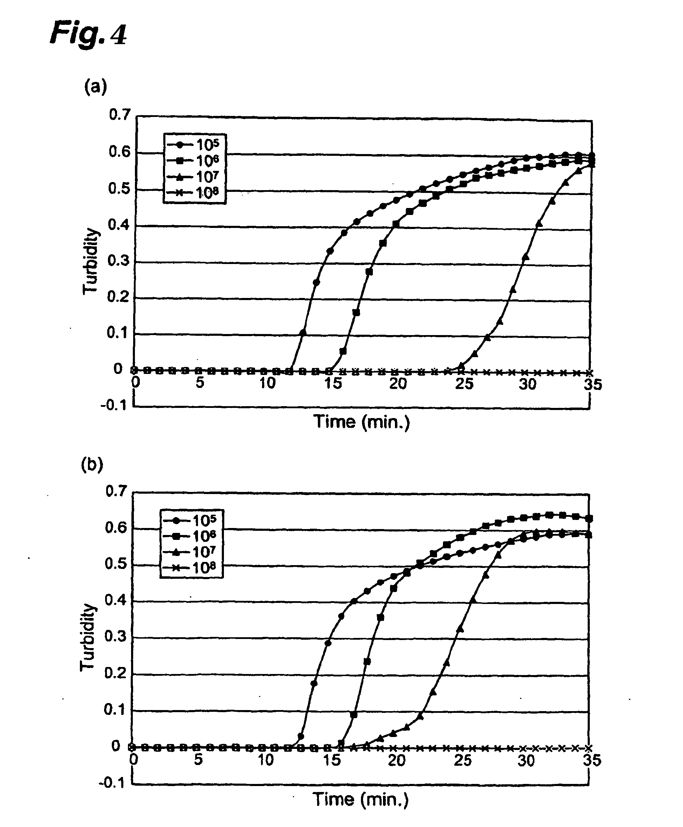

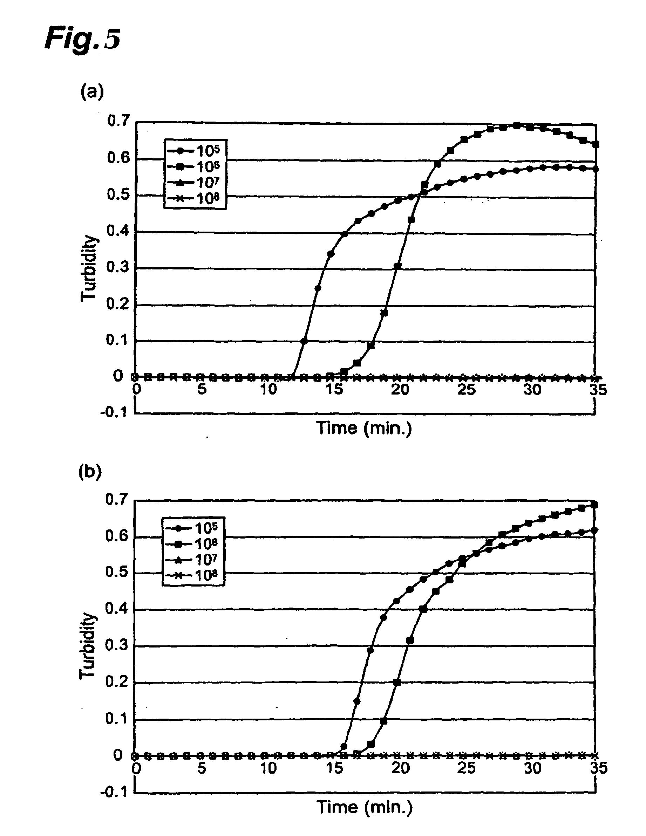

Figures 4 and 5 show a result of the real-time detection using each virus genome as a template sample. Up to 10⁷ diluents could be detected for A/Netherlands/219/2003 (H7N7) and A/Netherlands/33/2003 (H7N7) (see Figure 4), while up to 10⁶ diluents could be detected for A/mallard/Netherlands/12/00 (H7N3) and A/wigeon/Osaka/1/2001 (H7N7) (see Figure 5). These results demonstrated that the primer set of the present invention reacts with these 4 strains.

### Industrial Applicability

According to the present invention, an H7 avian influenza virus can be detected quickly with high sensitivity.

## Claims

1. An oligonucleotide primer set, **characterized by** being capable of amplifying a nucleotide sequence specific for the H7 avian influenza virus and consisting of an inner primer set comprising oligonucleotide primers comprising the following a) and b), and, an outer primer set comprising oligonucleotide primers comprising the following c) and d):
a) 5'-(a nucleotide sequence complementary to the nucleotide sequence of SEQ ID NO: 49)-(an arbitrary nucleotide sequence having 0 to 50 bases)-(the nucleotide sequence of SEQ ID NO: 50)-3';
b) 5'-(the nucleotide sequence of SEQ ID NO: 52)-(an arbitrary nucleotide sequence having 0 to 50 bases)-(a nucleotide sequence complementary to the nucleotide sequence of SEQ ID NO: 53)-3';
c) a nucleotide sequence of SEQ ID NO: 51; and
d) a nucleotide sequence of SEQ ID NO: 57.

2. The oligonucleotide primer set according to claim 1, wherein the inner primer set comprises oligonucleotide primers comprising the nucleotide sequence of SEQ ID NOs: 55 and 56.

3. The oligonucleotide primer set according to claim 2, further comprising a loop primer set comprising oligonucleotide primers comprising the nucleotide sequence of SEQ ID NOs: 58 and 59.

4. A method for detection of an H7 avian influenza virus, **characterized by** comprising performing an amplification reaction of a target nucleic acid region of an H7 avian influenza virus by use of an oligonucleotide primer set according to any one of Claims 1 to 3, wherein the amplification reaction is a LAMP method.

5. A method for influenza diagnosis, **characterized by** comprising detecting amplification of a target nucleic acid region of an H7 avian influenza virus by use of an oligonucleotide primer set according to any one of Claims 1 to 3 and thereby diagnosing the presence or absence of infection with the H7 avian influenza virus.

6. A kit for influenza diagnosis, **characterized by** comprising an oligonucleotide primer set according to any one of Claims 1 to 3.

## Patentansprüche

1. Oligonucleotidprimerset, **gekennzeichnet dadurch, dass** es fähig ist, eine für das H7 Vogelinfluenzavirus spezifische Nucleotidsequenz zu amplifizieren, und aus einem inneren Primerset, umfassend Oligonucleotidprimer, umfassend die folgenden a) und b), und einem äußeren Primerset, umfassend Oligonucleotidprimer, umfassend die folgenden c) und d), besteht:
a) 5' - (eine zu der Nucleotidsequenz der SEQ ID NO: 49 komplementäre Nucleotidsequenz) - (eine beliebige Nucleotidsequenz mit 0 bis 50 Basen) - (die Nucleotidsequenz der SEQ ID NO: 50) - 3';
b) 5' - (die Nucleotidsequenz der SEQ ID NO: 52) - (eine beliebige Nucleotidsequenz mit 0 bis 50 Basen) - (eine zu der Nucleotidsequenz der SEQ ID NO: 53 komplementäre Nucleotidsequenz) - 3';
c) eine Nucleotidsequenz der SEQ ID NO: 51 und
d) eine Nucleotidsequenz der SEQ ID NO: 57.

2. Oligonucleotidprimerset gemäß Anspruch 1, wobei das innere Primerset Oligonucleotidprimer umfasst, welche die Nucleotidsequenz der SEQ ID NO: 55 und 56 umfassen.

3. Oligonucleotidprimerset gemäß Anspruch 2, ferner umfassend ein Loop-Primerset, umfassend Oligonucleotidprimer, welche die Nucleotidsequenz der SEQ ID NO: 58 und 59 umfassen.

4. Verfahren zum Nachweisen eines H7 Vogelinfluenzavirus, **gekennzeichnet dadurch, dass** es das Durchführen einer Amplifikationsreaktion einer Ziel-Nucleinsäureregion eines H7 Vogelinfluenzavirus unter Verwendung eines Oligonucleotidprimersets gemäß einem der Ansprüche 1 bis 3 umfasst, wobei es sich bei der Amplifikationsreaktion um ein LAMP-Verfahren handelt.

5. Verfahren zur Influenza-Diagnose, **gekennzeichnet dadurch, dass** es das Nachweisen der Amplifikation einer Ziel-Nucleinsäureregion eines H7 Vogelinfluenzavirus unter Verwendung eines Oligonucleotidprimersets gemäß einem der Ansprüche 1 bis 3 und dadurch das Diagnostizieren der Anwesenheit oder Abwesenheit einer Infektion mit dem H7 Vogelinfluenzavirus umfasst.

6. Kit zur Influenza-Diagnose, **gekennzeichnet dadurch, dass** er ein Oligonucleotidprimerset gemäß einem der Ansprüche 1 bis 3 umfasst.

## Revendications

1. Jeu d'amorces oligonucléotidiques, **caractérisé en ce qu'**il est apte à amplifier une séquence nucléotidique spécifique du virus de la grippe aviaire H7 et constitué d'un jeu d'amorces internes comprenant des amorces oligonucléotidiques comprenant les a) et b) suivantes, et, d'un jeu d'amorces externes comprenant des amorces oligonucléotidiques comprenant les c) et d) suivantes :
a) 5'-(une séquence nucléotidique complémentaire de la séquence nucléotidique de SEQ ID NO: 49) - (une séquence nucléotidique arbitraire ayant 0 à 50 bases)-(la séquence nucléotidique de SEQ ID NO: 50)-3' ;
b) 5'-(la séquence nucléotidique de SEQ ID NO: 52)-(une séquence nucléotidique arbitraire ayant 0 à 50 bases)-(une séquence nucléotidique complémentaire de la séquence nucléotidique de SEQ ID NO: 53)-3' ;
c) une séquence nucléotidique de SEQ ID NO: 51 ; et
d) une séquence nucléotidique de SEQ ID NO: 57.

2. Jeu d'amorces oligonucléotidiques selon la revendication 1, dans lequel le jeu d'amorces internes comprend des amorces oligonucléotidiques comprenant la séquence nucléotidique de SEQ ID NOs: 55 et 56.

3. Jeu d'amorces oligonucléotidiques selon la revendication 2, comprenant en plus un jeu d'amorces boucles comprenant des amorces oligonucléotidiques comprenant la séquence nucléotidique de SEQ ID NOs: 58 et 59.

4. Procédé de détection d'un virus de la grippe aviaire H7, **caractérisé en ce qu'**il comprend la réalisation d'une réaction d'amplification d'une région d'acide nucléique cible d'un virus de la grippe aviaire H7 au moyen d'un jeu d'amorces oligonucléotidiques selon l'une quelconque des revendications 1 à 3, la réaction d'amplification étant une méthode LAMP (*Loop-mediated Isothermal Amplification*)*.*

5. Procédé de diagnostic d'une grippe, **caractérisé en ce qu'**il comprend la détection de l'amplification d'une région d'acide nucléique cible d'un virus de la grippe aviaire H7 au moyen d'un jeu d'amorces oligonucléotidiques selon l'une quelconque des revendications 1 à 3, et permet ainsi de diagnostiquer la présence ou l'absence d'infection par le virus de la grippe aviaire H7.

6. Trousse pour le diagnostic d'une grippe, **caractérisée en ce qu'**elle comprend un jeu d'amorces oligonucléotidiques selon l'une quelconque des revendications 1 à 3.
